(19) Europäisches Patentamt
European Patent Office
Office européen des brevets

(11) Veröffentlichungsnummer : **0 084 128**
**B1**

(12) # EUROPÄISCHE PATENTSCHRIFT

(45) Veröffentlichungstag der Patentschrift : **21.01.87**

(21) Anmeldenummer : **82111633.2**

(22) Anmeldetag : **15.12.82**

(51) Int. Cl.⁴ : **C 07 C 21/24**, C 07 C 17/26, C 07 C 17/20, C 07 D317/46, C 07 D319/20

(54) **Verfahren zur Herstellung von teilweise neuen Alkylbenzolen, die durch Fluor enthaltende Reste substituiert sind.**

(30) Priorität : **24.12.81 DE 3151365**

(43) Veröffentlichungstag der Anmeldung : **27.07.83 Patentblatt 83/30**

(45) Bekanntmachung des Hinweises auf die Patenterteilung : **21.01.87 Patentblatt 87/04**

(84) Benannte Vertragsstaaten : **BE CH DE FR GB IT LI**

(56) Entgegenhaltungen :
**EP-A- 0 008 453**
**EP-A- 0 041 131**
**GB-A- 2 070 012**
**BULLETIN OF THE CHEMICAL SOCIETY OF JAPAN, Band 49, Nr. 7, 1976 TOKYO K. TAMAO et al. Nickel-phosphine complex-catalyzed grignard coupling. I. cross-coupling of alkyl, aryl, and alkenyl grignard reagents with aryl and alkenyl halides: General scope and limitations "Seiten 1958, 1965-1969**
**J. Org. Chem. 44, 2907-2910(1979) Ullmann, Band II (4. Auflage ), Seite 634**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Marhold, Albrecht, Dr.**
**Carl-Duisberg-Strasse 329**
**D-5090 Leverkusen 1 (DE)**
Erfinder : **Klauke, Erich, Dr.**
**Eichendorffweg 8**
**D-5068 Odenthal (DE)**

**EP 0 084 128 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von teilweise neuen Alkylbenzolen, die durch Fluor enthaltende Reste substituiert sind, aus aromatischen Verbindungen.

Benzotrifluoride lassen sich nicht in üblicher Weise mit Hilfe eines Friedel-Crafts-Katalysators alkylieren, da hierbei in unerwünschter Weise vorrangig die Trifluormethylgruppe reagiert. Als Endprodukt erhält man im wesentlichen harzartige Produkte und Teer (J. Am. Chem. Soc. *60*, 1967 und 1968 (1938)). Diese Tatsache wird auch in Bull. Soc. Chim. France *1962*, 587-593 bestätigt, wo die Alkylierung von Benzotrifluorid analog der Chlormethylierung nach Friedel-Crafts untersucht wird.

Die Herstellung der wenigen bekannten Alkylbenzotrifluoride erfolgt daher nach speziellen Methoden. So ist aus J. Am. Chem. Soc. *65*, 389 (1943) die Herstellung von m-Methylbenzotrifluorid aus dem m-Brom-benzotrifluorid über eine Grignard-Reaktion bekannt.

Die Chlormethylierung von Benzotrifluorid wird in US 3 465 051 beschrieben. Abgesehen davon, daß man nach diesem Verfahren nur das Chlormethyl-benzotrifluorid erhält, das noch weiter zu der entsprechenden Alkylverbindung umgesetzt werden muß, entstehen als Nebenprodukte in unerwünschter Weise Dichlormethylether.

Aus der EP-A1 8 453 ist es bekannt, daß man durch Umsetzung aromatischer Verbindungen mit Tetrachlorkohlenstoff und Fluorwasserstoff in diese ein $CF_3$-Gruppe einführen kann. Bei dieser Reaktion handelt es sich im Prinzip um eine Acylierung, bei der pro Mol aromatischer Verbindung 3 Mole Fluorwasserstoff benötigt werden. Wenn eine $CF_3$-Gruppe eingeführt oder bereits vorhanden ist, findet keine Reaktion mehr statt. Aus der EP-A2 41 131 ist die Verbindung Trifluor-3,4-dioxyethylen-toluol bekannt, aus Bull. Chem. Soc. Jap. *49*, S. 1958 und 1965 bis 1969 (1976) die Verbindung 4-Butyl-1-trifluormethylbenzol. Beide Verbindungen wurden auf grundsätzlich andere Weise erhalten als bei der vorliegenden Erfindung.

Nach den bekannten Verfahren ist daher eine technische Herstellung von Alkylbenzotrifluoriden und auch anderer Alkylbenzole, die durch Fluor enthaltende Reste substituiert sind, nicht möglich.

Es wurde ein Verfahren zur Herstellung von aromatischen Verbindungen, die durch Fluor enthaltende Reste substituiert sind, der Formel

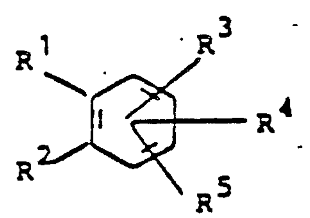

(I)

in der

$R^1$ für einen $CF_3$- oder $CF_3O$-Rest steht oder zusammen mit $R^2$ eine $—OCF_2O—$, $—O—CHF—CF_2O—$, $—O—CF_2CFCl—O—$ oder $—O—CF_2—CF_2—O$-Gruppe bildet,

$R^2$, $R^3$ und $R^4$ gleich oder verschieden sind und Wasserstoff, Halogen, Hydroxy, Alkyl, Aryl, Alkoxy oder Aroxy bedeuten, und

$R^5$ für einen gegebenenfalls substituierten Alkylrest steht,

gefunden, das dadurch gekennzeichnet ist, daß man aromatische Verbindungen der Formel

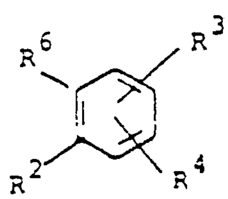

(II)

in der

$R^6$ für einen $CF_3$-, $CF_2Cl$-, $CFCl_2$-, $CCl_3$- oder $CF_3O$-Rest steht oder zusammen mit $R^2$ eine $—OCF_2O$, $—O—CHF—CF_2—O—$, $—O—CF_2—CFCl—O—$ oder $—O—CF_2—CF_2—O$-Gruppe bildet, und

$R^2$, $R^3$ und $R^4$ die obengenannte Bedeutung haben,

mit einem Alkylierungsmittel in Gegenwart von Fluorwasserstoff im Temperaturbereich von 0 bis 150 °C umsetzt.

Durch das erfindungsgemäße Verfahren sind eine Reihe von aromatischen Verbindungen, die durch Fluor enthaltende Kohlenwasserstoffreste substituiert sind, insbesondere Alkylbenzotrifluoride, einer technischen Herstellung zugänglich.

Das erfindungsgemäße Verfahren kann durch die folgende Reaktionsgleichung erläutert werden :

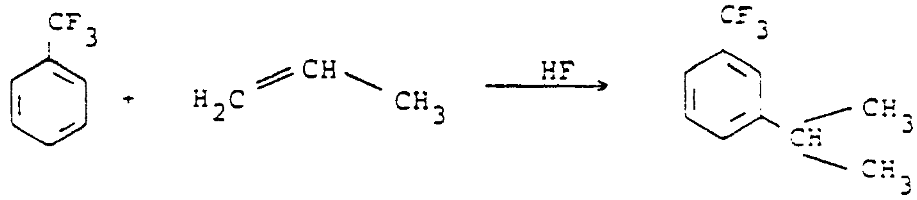

Bevorzugt steht $R^1$ für Trifluormethyl und $R^6$ für Trifluormethyl, Difluorchlormethyl und Trichlormethyl.

Alkyl bedeutet im allgemeinen erfindungsgemäß ein geradkettiger oder verzweigter Kohlenwasserstoffrest mit 1 bis 12 Kohlenstoffatomen, bevorzugt ein Niederalkylrest mit 1 bis etwa 6 Kohlenstoffatomen. Beispielsweise seien die folgenden Alkylreste genannt : Methyl, Ethyl, Propyl, Isopropyl, Butyl, Isobutyl, Pentyl, Isopentyl, Hexyl und Isohexyl.

Aryl bedeutet im allgemeinen ein aromatischer Rest, bevorzugt Phenyl, Naphthyl und Biphenyl.

Alkoxy bedeutet im allgemeinen erfindungsgemäß ein über Sauerstoff verbundener geradkettiger oder verzweigter Kohlenwasserstoffrest mit 1 bis 12 Kohlenstoffatomen, bevorzugt ein Niederalkoxyrest mit 1 bis etwa 6 Kohlenstoffatomen. Beispielsweise seien die folgenden Alkoxyreste genannt : Methoxy, Ethoxy, Propoxy, Isopropoxy, Butoxy, Isobutoxy, Pentoxy, Isopentoxy, Hexoxy und Isohexoxy.

Aroxy bedeutet im allgemeinen erfindungsgemäß ein über Sauerstoff verbundener aromatischer Rest, bevorzugt Phenoxy, Naphthoxy und Phenylphenoxy.

Die Reste $R^2$ bis $R^5$, insbesondere der Rest $R^5$, können durch weitere Reste substituiert sein, die sich unter den Reaktionsbedingungen nicht verändern. Beispielsweise seien hier die Halogene, insbesondere Fluor, Chlor und Brom, genannt.

Unter den erfindungsgemäßen Reaktionsbedingungen werden alle Halogenatome des Restes $R^6$ mit Fluor ausgetauscht.

Die aromatischen Verbindungen für das erfindungsgemäße Verfahren sind an sich bekannt (Houben-Weyl, Band 5/3, Georg Thieme Verlag, Stuttgart).

Verbindungen in denen die Reste $R^2$ und $R^6$ durch eine Etherbrücke verbunden sind, können hergestellt werden, indem man Benzodioxole halogeniert oder Brenzkatechin mit Perhalogenethylenen (z. B. Trifluorchlorethylen) kondensiert und gegebenenfalls noch nachträglich halogeniert (z. B. chloriert).

Bevorzugte aromatische Verbindungen zum Einsatz in das erfindungsgemäße Verfahren sind Verbindungen der Formel

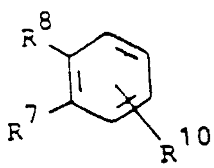

(III)

worin

$R^8$ für $CF_3$, $CF_2Cl$ oder $OCF_3$ und

$R^7$ für Wasserstoff steht

oder worin

$R^7$ und $R^8$ gemeinsam eine $-OCF_2O-$, $-OCHF-CF_2-$, $-OCF_2-CF_2O-$-Gruppe bilden, und

$R^{10}$ für Wasserstoff oder Niederalkyl steht.

Beispielsweise seien die folgenden aromatischen Verbindungen genannt : Benzotrifluorid, Difluorchlormethylbenzol, die isomeren Methylbenzotrifluoride, Difluorbenzodioxol und 2,2,3-Trifluorbenzodioxen-1,4.

Als Alkylierungsmittel für das erfindungsgemäße Verfahren kommen die für Friedel-Crafts-Alkylierungen üblichen Alkylierungsmittel in Frage.

Bevorzugt für das erfindungsgemäße Verfahren werden als Alkylierungsmittel Alkylhalogenide, Alkohole, Ether, Alkylaromaten oder Halogenameisensäurealkylester der Formel

$$A—R^5 \qquad (IV),$$

worin

$R^5$ die obengenannte Bedeutung hat und

A für Halogen, Hydroxy, Alkoxy, Aryl oder Halogenformyl steht,

oder Olefine mit einer endständigen Doppelbindung, wobei das Olefin in der Anzahl der Kohlenstoffatome $R^5$ entspricht.

Im besonderen bevorzugt werden als Alkylierungsmittel Alkylhalogenide, Alkylaromaten oder

Halogenameisensäurealkylester der Formel

R—$R^5$

worin

$R^1$ die obengenannte Bedeutung hat und

B für Halogen, Aryl, Halogenformyl steht,

oder Olefine mit einer endständigen Doppelbindung, wobei das Olefin in der Anzahl der Kohlenstoffatome $R^5$ entspricht.

Beispielsweise seien die folgenden Alkylierungsmittel genannt : Methylfluorid, Methylchlorid, Methylbromid, Methyljodid, Ethylchlorid, Ethylbromid, Isopropylfluorid, Isopropylchlorid, Isopropylbromid, n-Propylchlorid, n-Propyljodid, die isomeren Butylchloride, n-Butylbromid, Hexylchlorid, Octylbromid, Ethylen, Propen, die isomere Butene, Penten, Hexen, Chlorameisensäuremethylester, Chlorameisensäureethylester, Chlorameisensäureisopropylester, Ethylbenzol, Diethylbenzol, Isopropylbenzol, Diisopropylbenzol, Mesitylen, Trimethylbenzotrifluorid, Diisopropylbenzotrifluorid, Nitrobenzylchlorid, 2-, 3- und 4-Trifluormethylbenzylchlorid, 2-Chlor-5-trifluormethylbenzylchlorid, Isopropanol, tert.-Butanol, Cyclohexanol, Diisopropylether, 3-Trifluormethyl-benzyl-methylether.

Das erfindungsgemäße Verfahren wird in Gegenwart von Fluorwasserstoff durchgeführt. Es ist möglich, für das erfindungsgemäße Verfahren den Fluorwasserstoff in technischer Reinheit einzusetzen. Vorzugsweise verwendet man für das erfindungsgemäße Verfahren einen Fluorwasserstoff der möglichst wasserfrei ist. Es ist besonders bevorzugt, für das erfindungsgemäße Verfahren einen Fluorwasserstoff zu verwenden, der weniger als 1 % Wasser enthält.

Die Menge des Alkylierungsmittels ist selbstverständlich abhängig von der Anzahl der Alkylreste, die in die aromatische Verbindung eingeführt werden sollen. Üblicherweise setzt man 0,1 bis 4 Mol, bevorzugt 1 bis 3 Mol, des Alkylierungsmittels, bezogen auf die aromatische Verbindung, ein.

Der Fluorwasserstoff kann sowohl in kleinen Mengen als auch in einem großen Überschuß eingesetzt werden. Zur Durchführung des erfindungsgemäßen Verfahrens ist es vorteilhaft, wenn so viel Fluorwasserstoff eingesetzt wird, daß zwei getrennte Phasen im Reaktionsgemisch entstehen. Hierzu ist es im allgemeinen ausreichend 1 bis 20 Mol, bevorzugt 2 bis 10 Mol, des Fluorwasserstoffs, bezogen auf 1 Mol der aromatischen Verbindung, einzusetzen. Bei Verwendung eines größeren Überschusses an Fluorwasserstoff wird er als Lösungs- bzw. Verdünnungsmittel eingesetzt. Das erfindungsgemäße Verfahren wird im allgemeinen im Temperaturbereich von — 20 bis 200 °C, bevorzugt von 0 bis 160 °C, durchgeführt.

Das erfindungsgemäße Verfahren wird im allgemeinen unter Normaldruck (etwa 1 bar) oder Überdruck (bis zu 300 bar) durchgeführt. Da das erfindungsgemäße Verfahren in flüssiger Phase durchgeführt wird, ist bei höheren Temperaturen ein höherer Druck zweckmäßig, damit der Fluorwasserstoff in flüssiger Form vorliegt. Ein Überdruck ist auch dann zweckmäßig, wenn das Alkylierungsmittel ein niedrigen Siedepunkt besitzt oder eine höhere Löslichkeit der Reaktionspartner ineinander gewünscht wird.

Der gewünschte Druck für das erfindungsgemäße Verfahren stellt sich im allgemeinen bei einer Arbeit im Autoklaven durch den Eigendruck des Reaktionsgemisches ein. Es ist aber auch möglich, den gewünschten Druck durch Aufdrücken eines inerten Gases, beispielsweise Stickstoff, einzustellen.

Es ist auch möglich, das erfindungsgemäße Verfahren in Gegenwart eines Lösungs- bzw. Verdünnungsmittels durchzuführen. Als Lösungs- bzw. Verdünnungsmittel für das erfindungsgemäße Verfahren kommen alle Zusätze in Frage, die sich unter den erfindungsgemäßen Reaktionsbedingungen nicht verändern. Beispielsweise seien genannt : Dichlormethan, Tetrachlormethan und Fluortrichlormethan.

Das erfindungsgemäße Verfahren kann beispielsweise wie folgt durchgeführt werden :

Die Reaktionskomponenten werden in beliebiger Reihenfolge in das Reaktionsgefäß gegeben. So ist es möglich, den Katalysator zuerst vorzulegen und die durch Fluor enthaltende Reste substituierte aromatische Verbindung zuerst zu mischen und dann das Alkylierungsmittel hinzuzugeben. Es ist ebenso möglich, den Katalysator zuletzt zuzudosieren. Weiterhin ist es möglich, die Reaktionskomponenten einschließlich dem Katalysator, gasförmig, flüssig oder in festem Zustand miteinander zu mischen bzw. in Kontakt zu bringen.

Das Reaktionsgemisch wird gegebenenfalls unter Druck auf die gewünschte Reaktionstemperatur gebracht. Nach Beendigung der Reaktion trennt man die alkylierten aromatischen Verbindungen, die durch Fluor enthaltende Reste substituiert sind, in an sich bekannter Weise, beispielsweise durch Destillation, ab.

Nach dem erfindungsgemäßen Verfahren erhält man in der Regel ein Isomerengemisch der alkylierten Verbindung. Im Falle einer Monoalkylierung erhält man, falls es möglich ist, bevorzugt das para- und das meta-substituierte Alkylierungsprodukt.

Die einzelnen Isomeren können durch Destillation voneinander getrennt werden.

Da übliche Alkylierungsmethoden nicht zu einem Erfolg bei der Alkylierung von aromatischen Verbindungen, die durch Fluor enthaltende Reste substituiert sind, führen, ist es überraschend, daß mit Hilfe des erfindungsgemäßen Verfahrens eine Alkylierung in einfacher Weise ermöglicht wird.

Da aus Kirk-Othmer, Encyclopädia of Chemical Technology III Ed. Vol. 2, S. 58 bekannt ist, daß Fluorwasserstoff als Katalysator für die Alkylierung von Aromaten mit Olefinen nicht brauchbar ist, ist die erfindungsgemäße Verwendung von Fluorwasserstoff besonders überraschend.

Die erfindungsgemäß hergestellten aromatischen Verbindungen, die durch Fluor enthaltende Reste substituiert sind, sind teilweise neu.

Bevorzugte neue aromatische Verbindungen, die durch Fluor enthaltende Reste substituiert sind, sind Verbindungen der folgenden Formeln :

a)

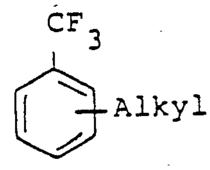

(V)

wobei, für den Fall, daß der Alkylrest in 4-Stellung substituiert ist, dieser aus einem geradkettigen oder verzweigten Kohlenwasserstoffrest mit 3 bis 6 Kohlenstoffatomen besteht oder für den Fall, daß der Alkylrest in 2- oder 3-Stellung substituiert ist, dieser aus einem geradkettigen oder verzweigten Alkylrest mit 4 bis 6 Kohlenstoffatomen besteht, mit Ausnahme der Verbindung 4-Butyl-1-trifluormethylbenzol.

b)

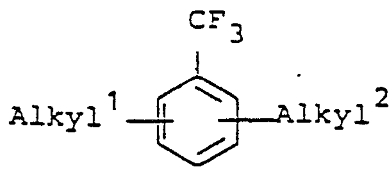

(VI)

wobei Alkyl[1] und Alkyl[2] gleich sind und für einen geradkettigen oder verzweigten Kohlenwasserstoffrest mit 3 bis 6 Kohlenstoffatomen stehen oder verschieden sind, wobei Alkyl[1] für Methyl oder Ethyl steht und Alkyl[2] für einen geradkettigen oder verzweigten Kohlenwasserstoffrest mit 3 bis 6 Kohlenstoffatomen,

c)

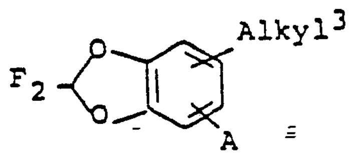

(VII)

wobei für den Fall, daß A Wasserstoff bedeutet, Alkyl[3] für einen geradkettigen oder verzweigten Kohlenwasserstoffrest mit 4 bis 6 Kohlenstoffatomen steht, oder für den Fall, daß A Halogen, Niederalkyl oder Niederalkoxy bedeutet, Alkyl[3] für einen Niederalkylrest steht, und

d)

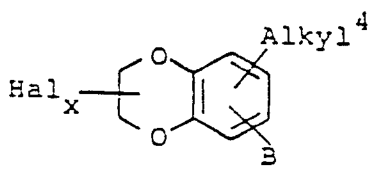

(VIII)

wobei

Hal für Halogen steht,

X eine Zahl von 1 bis 4 bedeutet,

Alkyl[4] für den Fall, daß B für Wasserstoff steht, einen geradkettigen oder verzweigten Kohlenwasserstoffrest bedeutet und für den Fall daß B für Halogen, Niederalkyl oder Niederalkoxy steht, Niederalkyl bedeutet,

mit Ausnahme der Verbindung Trifluor-3,4-dioxyethylentoluol.

Die nach dem erfindungsgemäßen Verfahren hergestellten aromatischen Verbindungen, die durch Fluor enthaltende Reste substituiert sind, sind Zwischenprodukte für Pflanzenschutzmittel. So kann aus dem 2-Ethyl-benzotrifluorid durch Umsetzung mit Salpetersäure, Reduktion der Nitroverbindung zum Amin und Reaktion mit Dimethylcarbamidsäurechlorid der 1-(3-Trifluormethyl-4-ethyl)phenyl-3,3-dimethylharnstoff hergestellt werden, der als Herbizid verwendet wird (GB 2 077 609).

Beispiel 1

In einem gegen Fluorwasserstoff beständigen Autoklaven werden bei 0 °C 500 ml Fluorwasserstoff vorgelegt und eine Mischung von 2 000 g Benzotrifluorid und 1 000 g Isopropylchlorid zugegeben. Nach Aufdrücken von 3 bar Stickstoff wird für 6 Stunden bei 50 °C gerührt und der entstehende Chlorwasserstoff bei 8 bar kontinuierlich entspannt. Nach Abkühlen wird der Fluorwasserstoff abdestilliert und der Rückstand (2 430 g) durch Gaschromatographie analysiert.

Es sind enthalten :

8,6 % Isopropylchlorid
38,3 % Benzotrifluorid
24,2 % 3-Isopropylbenzotrifluorid
5,8 % 4-Isopropylbenzotrifluorid
15,3 % 3,5-Diisopropylbenzotrifluorid
6,3 % 2,5-Diisopropylbenzotrifluorid

Durch Destillation können die Reaktionsprodukte voneinander getrennt werden. Sie haben folgende physikalische Daten :

3-Isopropylbenzotrifluorid Kp : 53 °C/16 mbar $n_D^{20}$ 1.436 0
4-Isopropylbenzotrifluorid Kp : 55-56 °C/14 mbar $n_D^{20}$ 1.437 0
3,5-Diisopropylbenzotrifluorid Kp : 87 °C/15 mbar $n_D^{20}$ 1.448 5
2,5-Diisopropylbenzotrifluorid Kp : 90 °C/17 mbar $n_D^{20}$ 1.453 0

Beispiel 2

In einem auf — 50 °C gekühlten VA-Autoklaven werden 500 ml Fluorwasserstoff, 146 g Benzotrifluorid und 50 g Isopropylfluorid eingebracht. Die Reaktionsmischung wird für 5 Stunden bei 60 °C gerührt, der Fluorwasserstoff abdestilliert und die Reaktionsmischung analysiert. Der Anteil an 3-Isopropylbenzotrifluorid beträgt 34,5 %.

Beispiel 3

Eine Mischung aus 146 g Benzotrifluorid, 50 g Fluorwasserstoff und 70 g Isopropylchlorid wird für 6 Stunden bei 16 °C gerührt. Die gaschromatographische Analyse der organischen Phase zeigt folgende Zusammensetzung :

25 % Isopropylchlorid
54 % Benzotrifluorid
15,1 % 3-Isopropylbenzotrifluorid
1,2 % 4-Isopropylbenzotrifluorid
2,6 % 3.5-Diisopropylbenzotrifluorid
1,5 % 2,5-Diisopropylbenzotrifluorid

Der Umsatz an Benzotrifluorid beträgt 56 %, die Selektivität bezogen auf 3-Isopropylbenzotrifluorid beträgt 72 %.

Beispiel 4

Erhitzt man eine Mischung von 146 g Benzotrifluorid, 68 g Isopropylchlorid und 500 ml Fluorwasserstoff für 6 Stunden auf 50 °C und stellt einen Druck von 20 bar durch Aufpressen von Stickstoff ein, so erhält man ein Reaktionsgemisch, das zu 48,3 % aus mono-Isopropylbenzotrifluoriden besteht.

Beispiel 5

Wiederholt man den Versuch aus Beispiel 4 und setzt 250 ml Dichlormethan zu, so enthält die Reaktionsmischung 26,2 % 3-Isopropylbenzotrifluorid neben 7,4 % 4-Isopropylbenzotrifluorid und 23,3 % 3,5-Diisopropylbenzotrifluorid.

Beispiel 6

In einem VA-Autoklaven werden 300 ml Fluorwasserstoff und 146 g Benzotrifluorid bei — 5 °C vorgelegt und unter Rühren 50 g Propen aufgedrückt. Die Temperatur der Reaktionsmischung steigt exotherm auf 12 °C. Dann werden noch 3 bar Stickstoff aufgedrückt und anschließend für 5 Stunden bei 60 °C gerührt. Nach Abdestillieren der Flußsäure verbleibt ein Gemisch, das nach gaschromatographischer Analyse zu 45,8 % aus Benzotrifluorid, 26,3 % 3-Isopropylbenzotrifluorid 5,4 % 4-Isopropylbenzotrifluorid und 10,9 % 3,5-Diisopropylbenzotrifluorid besteht.

Beispiel 7

In einem VA-Autoklaven wird eine Mischung von 200 ml Fluorwasserstoff, 50 g Benzotrifluorid und 20 g 3,5-Diisopropylbenzotrifluorid unter Rühren für 5 Stunden auf 60 °C unter Eigendruck erhitzt. Nach Abdestillieren des Fluorwasserstoffs wird das Reaktionsgemisch analysiert. Der Umsatz von Diisopropylbenzotrifluorid beträgt etwa 30 % und die Selektivität für 3-Isopropylbenzotrifluorid 91 %.

Beispiel 8

In einem VA-Autoklaven werden 300 ml Fluorwasserstoff vorgelegt und eine Mischung aus 146 g Benzotrifluorid und 100 g Chlorameisensäureethylester zugegeben. Dann drückt man 3 bar Stickstoff auf und erhitzt unter Rühren für 6 Stunden auf 100 °C, wobei das entstehende Gasgemisch bei 14 bar kontinuierlich über einen Rückflußkühler entspannt wird. Destillative Aufarbeitung liefert 76 g einer Mischung aus 2-, 3- und 4-Ethylbenzotrifluorid. Kp. : 98-102 °C/200 mbar, $n_D^{20}$ 1.437 2.

Beispiel 9

Analog Beispiel 6 werden 146 g Benzotrifluorid mit 30 g Ethylen in 200 ml Fluorwasserstoff in 7 Stunden bei 75 °C umgesetzt. Die organische Phase enthält neben 51,1 % unumgesetzten Benzotrifluorid 21,2 % 3-Ethylbenzotrifluorid und 17,7 % 4-Ethylbenzotrifluorid.

Beispiel 10

In einem VA-Autoklaven werden 1 Mol (146 g) Benzotrifluorid mit 0,5 Mol Methyljodid und 200 ml Fluorwasserstoff für 7 Stunden bei 140 °C unter Eigendruck umgesetzt. Die Analyse der Reaktionsmischung zeigt einen Umsatz bezogen auf Benzotrifluorid von 8,4 % und die Bildung von 3-Methylbenzotrifluorid mit einer Selektivität von 42 %.

Beispiel 11

Wiederholt man Beispiel 10 mit Methylbromid anstelle von Methyljodid, so beträgt der Anteil an unumgesetztem Benzotrifluorid 92,2 % und der Anteil an 3-Methylbenzotrifluorid 1,1 % und an Dimethylbenzotrifluorid 1,7 % am Reaktionsgemisch (ohne Fluorwasserstoff und nicht umgesetztes Methylbromid).

Beispiel 12

Analog Beispiel 10 werden 146 g Benzotrifluorid mit 100 g Chlorameisensäuremethylester in 500 ml Fluorwasserstoff in 5 Stunden bei 115 °C umgesetzt. Die Ausbeute an 3-Methylbenzotrifluorid beträgt 82 % bezogen auf den Umsatz von Benzotrifluorid.

Beispiel 13

Man legt in einem VA-Autoklaven mit Rückflußkühler 500 ml Fluorwasserstoff bei 0 °C vor und tropft eine Mischung aus 100 g Benzotrifluorid und 40 g Isopropylchlorid zu. Es setzt sofort die Entwicklung von Chlorwasserstoff ein und man rührt bis Ende dieser Gasentwicklung bei 20 °C (ca. 10 Stunden). Man gewinnt überschüssigen Fluorwasserstoff durch Destillation zurück und analysiert das rohe Reaktionsprodukt. Neben 15 % Benzotrifluorid sind 42,5 % 3-Isopropylbenzotrifluorid, 7,9 % 4-Isopropylbenzotrifluorid, 20,2 % 3,5-Diisopropylbenzotrifluorid und 11,7 % 2,5-Diisopropylbenzotrifluorid entstanden.

Beispiel 14

Eine Mischung aus 300 ml Fluorwasserstoff, 100 g 2-Methylbenzotrifluorid und 55 g Isopropylchlorid wird für 6 Stunden bei 20 °C gerührt und anschließend der Fluorwasserstoff abdestilliert. Durch fraktionierte Destillation werden bei Kp. : 64-67 °C/12 mbar 67 g 2-Methyl-5-isopropyl-benzotrifluorid ($n_D^{20}$ 1.448 8) und 15 g 2-Methyl-3,6-diisopropyl-benzotrifluorid ($n_D^{20}$ 1.462 9, Kp. : 102 °C/18 mbar) erhalten.

Beispiel 15

Erhitzt man eine Mischung aus 100 ml Fluorwasserstoff, 50 g 2-Chlor-5-methyl-benzotrifluorid und 20

g Isopropylchlorid für 5 Stunden auf 60 °C, so erhält man eine 12,8 %ige Umsetzung zu einem x-Isopropyl-2-chlor-5-methyl-benzotrifluorid.

Beispiel 16

In einem VA-Autoklaven werden 500 ml Fluorwasserstoff, 100 g Benzotrifluorid und 50 g 4-Nitrobenzylchlorid für 5 Stunden auf 120 °C bei einem Druck von 18,5 bis 20 bar erhitzt. Nach Abdestillieren des Fluorwasserstoffs und des überschüssigen Benzotrifluorids werden bei Kp. : 148-150 °C/0,4 mbar 66 g 4-Nitro-3'-trifluormethyl-diphenylmethan erhalten. Die Ausbeute bezogen auf Alkylierungsmittel beträgt ca. 80 % der Theorie.

Beispiel 17

Eine Mischung aus 500 ml Fluorwasserstoff, 146 g Benzotrifluorid und 100 g 1,3-Diisopropylbenzol wird für 3 Stunden bei 60 °C gerührt, dann die Flußsäure abdestilliert und die Reaktionsmischung gaschromatographisch analysiert. Es zeigt sich, daß 21 % Benzotrifluorid alkyliert wurden und 3-Isopropylbenzotrifluorid mit einer Selektivität von ca. 98 % bezogen auf Benzotrifluorid entstanden ist.

Beispiel 18

In 300 ml Fluorwasserstoff bei 0 °C wird eine Mischung aus 50 g Isopropylchlorid und 100 g 3-Hydroxybenzotrifluorid getropft. Man rührt bis Ende der Chlorwasserstoff-Entwicklung bei 20 °C nach und destilliert anschließend den Fluorwasserstoff ab. Durch fraktionierte Destillation der Reaktionsmischung wird neben unumgesetztem Ausgangsmaterial bei Kp. : 55-56 °C/0,03 mbar 6-Isopropyl-3-hydroxy-benzotrifluorid erhalten.

Beispiel 19

In ein auf 0 °C gekühltes VA-Reaktionsgefäß werden 300 ml Fluorwasserstoff eingebracht, danach 100 g Benzotrifluorid zugegeben und anschließend 60 g Octen-1 zugetropft. Man rührt für 2 Stunden bei 20 °C und noch 2 Stunden bei 50 °C/6 bar, kühlt ab und destilliert den Fluorwasserstoff ab. Durch fraktionierte Destillation werden neben unumgesetztem Benzotrifluorid 82 g 2-(3-trifluormethyl-phenyl)-octan ; Kp. : 80-85 °C/0,1 mbar ($n_D^{20}$ 1.461 5) erhalten.

Beispiel 20

Zu 146 g Benzotrifluorid und 300 ml Fluorwasserstoff tropft man bei 0 °C 76 g Allylchlorid, drückt 3 bar Stickstoff auf und rührt unter Eigendruck der Mischung für 5 Stunden bei 50 °C.

Nach Destillation lassen sich bei Kp. : 46-53 °C/0,1 mbar zwei isomere Monoalkylylierungsprodukte abtrennen, die die Struktur von 2-(x-Trifluormethylphenyl)-1-chlorpropan haben.

Beispiel 21

In einem VA-Autoklaven werden 300 ml Fluorwasserstoff und 56 g 4-Nitro-4'-trifluormethyldiphenylether bei 10 °C vorgelegt und 30 g Isopropylchlorid zugetropft. Man rührt zuerst für 1 Stunde bei 20 °C und dann unter Eigendruck für 3 Stunden bei 50 °C. Nach Abdestillieren des Fluorwasserstoffs wird das Reaktionsgemisch einer Analyse unterworfen, die zeigt, daß neben mono-Isopropylprodukt auch di-Isopropylprodukt vorliegt (Verhältnis 1 : 1).

Beispiel 22

In einer VA-Rührapparatur werden 200 ml Fluorwasserstoff bei 10 °C vorgelegt und nacheinander 100 g Benzotrifluorid und 40 g Isopropanol zugetropft. Dann wird für 2 Stunden bei 20 °C gerührt, auf Eis gegossen und die organische Phase abgetrennt. Die Reaktionsmischung hat laut GC-Analyse folgende Zusammensetzung : 24,4 % Benzotrifluorid, 36,8 % 3-Isopropylbenzotrifluorid, 3,4 % 4-Isopropylbenzotrifluorid, 20,2 % 3,5-Diisopropylbenzotrifluorid, 11,2 % 2,5-Diisopropylbenzotrifluorid und 2,7-5 % einer unbekannten Substanz.

Beispiel 23

In einem VA-Autoklaven werden 100 ml Fluorwasserstoff, 100 g Diisopropylether und 146 g Benzotrifluorid für 2 Stunden bei einem Stickstoffdruck von 6 bar auf 50 °C erhitzt. Nach dem Abkühlen wird der Fluorwasserstoff bei leichtem Vakuum abgezogen und der Rückstand mit Wasser gewaschen. Die getrocknete organische Phase wird nach GC analysiert. Es sind 57,75 % Benzotrifluorid, 26,2 % 3-Isopropylbenzotrifluorid, 1,9 % 4-Isopropylbenzotrifluorid und 8,4 % Diisopropylbenzotrifluorid enthalten.

Beispiel 24

In einer VA-Rührapparatur werden 200 ml Fluorwasserstoff und 292 g Benzotrifluorid unter intensivem Rühren vorgelegt. Dann werden innerhalb 2 Stunden 100 g Propen eingeleitet und anschließend 1 Stunde bei 17 bis 20 °C nachgerührt. Nach Abdestillieren des Fluorwasserstoffs verbleiben 378 g Reaktionsprodukt, das nach GC-Analyse 131,5 g Benzotrifluorid, 126,3 g 3-Isopropylbenzotrifluorid, 12,5 g 4-Isopropylbenzotrifluorid, 14,4 % 3,5-Diisopropylbenzotrifluorid, 6,6 % 2,5-Diisopropylbenzotrifluorid und 28,3 g Rückstand enthält. Daraus ergibt sich ein Verlust an Benzotrifluoriden von 2 % und ein Umwandlungsgrad von Propen von 60 %.

Beispiel 25

In einem VA-Autoklaven werden 100 ml Fluorwasserstoff, 36 g Trifluormethylphenylether und 20 g Chlorameisensäuremethylester unter 3 bar Stickstoff für 3 Stunden auf 60 °C und anschließend für 3 Stunden auf 100 °C erhitzt. Nach Abkühlen wird entspannt, Fluorwasserstoff abdestilliert und der organische Anteil einer GC-Analyse unterworfen. Neben 83,7 % Ausgangsmaterials sind 13,9 % 4-Methylphenyl-trifluormethylether und 2 % Xylyl-trifluormethylether entstanden.

Beispiel 26

Eine Mischung aus 100 ml Fluorwasserstoff, 158 g 2,2-Difluorbenzodioxol und 80 g Isopropylchlorid wird bei 0 bis 10 °C für 7 Stunden gerührt. Nach Abziehen des Fluorwasserstoffs wird die organische Phase destilliert. Man erhält 68 g 2,2-Difluor-5-isopropylbenzodioxol ; Kp. : 79-81 °C/12 mbar, $n_D^{20}$ 1.456 0.

Beispiel 27

Eine Mischung von 40 ml Fluorwasserstoff, 20 g 3-Trifluormethyl-benzyl-methylether und 30 g Benzotrifluorid wird für 3 Stunden bei 20 °C gerührt, auf Eis gegeben, die organische Phase abgetrennt, getrocknet und destilliert. Man erhält 25 g Bis(3-trifluormethyl-phenyl)-methan (Kp : 145-150 °C/22 mbar, F : 40-2 °C).

**Patentansprüche**

1. Verfahren zur Herstellung von aromatischen Verbindungen, die durch Fluor enthaltende Reste substituiert sind, der Formel

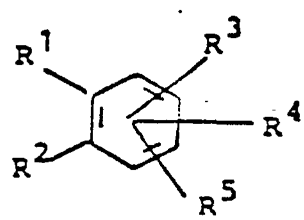

in der

$R^1$ für einen $CF_3$- oder $CF_3O$-Rest steht oder zusammen mit $R^2$ eine $-OCF_2O-$, $-O-CHF-CF_2-O-$, $-O-CF_2CFCl-O-$ oder $-O-CF_2-CF_2-O-$Gruppe bildet.

$R^2$, $R^3$ und $R^4$ gleich oder verschieden sind und Wasserstoff, Halogen, Hydroxy, Alkyl, Aryl, Alkoxy oder Aroxy bedeuten, und

$R^5$ für einen gegebenenfalls substituierten Alkylrest steht,

dadurch gekennzeichnet, daß man aromatische Verbindungen der Formel

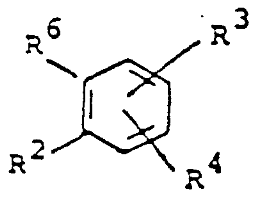

in der

$R^6$ für einen $CF_3$-, $CF_2Cl$-, $CCl_2F$-, $CCl_3$- oder $CF_3O$-Rest steht oder zusammen mit $R^2$ eine $-OCF_2O-$, $-O-CHF-CF_2-O-$, $-O-CF_2CFCl-O-$ oder $-O-CF_2-CF_2-O$-Gruppe bildet, und $R^2$, $R^3$ und $R^4$ die obengenannte Bedeutung haben,

mit einem Alkylierungsmittel in Gegenwart von Fluorwasserstoff im Temperaturbereich von 0 bis 150 °C umsetzt.

2. Verfahren nach Anspruch 1, dadurch gekennzeichnet, daß man als Alkylierungsmittel ein Alkylhalogenid, Alkohol, Ether, ein Olefin, einen Alkylaromaten oder einen Halogenameisensäurealkylester verwendet.

3. Verfahren nach den Ansprüchen 1 und 2, dadurch gekennzeichnet, daß man den Fluorwasserstoff in einer größeren Menge einsetzt, als die Menge des nicht als Fluor vorliegenden Halogens entspricht, das bei der Umsetzung durch Fluor ersetzt werden soll.

4. Verfahren nach den Ansprüchen 1 und 2, dadurch gekennzeichnet, daß man bei dem Einsatz von aromatischen Verbindungen der Formel

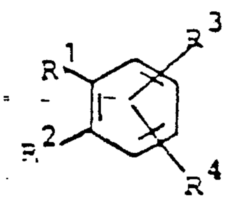

in der $R^1$, $R^2$, $R^3$ und $R^4$ die in Anspruch 1 genannte Bedeutung haben,

den Fluorwasserstoff in einer Menge einsetzt, die ausreichend ist, um im Reaktionsgemisch eine getrennte Phase zu bilden.

5. Verfahren nach den Ansprüchen 1 bis 4, dadurch gekennzeichnet, daß man das Alkylierungsmittel in einer Menge von 0,1 bis 4 Mol, bezogen auf 1 Mol der aromatischen Verbindung, einsetzt.

6. Verfahren nach den Ansprüchen 1 bis 5, dadurch gekennzeichnet, daß man das Alkylierungsmittel im Unterschuß, bezogen auf die aromatische Verbindung, einsetzt und die nicht-umgesetzte aromatische Verbindung einer weiteren Alkylierung zuführt.

7. Neue durch Fluor enthaltende Gruppen substituierte aromatische Verbindungen der Formel

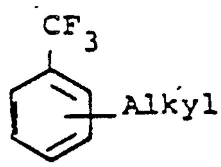

wobei für den Fall, daß der Alkylrest in 4-Stellung substituiert ist, dieser aus einen geradkettigen oder verzweigten Kohlenwasserstoffrest mit 3 bis 6 Kohlenstoffatomen besteht oder für den Fall, daß der Alkylrest in 2- oder 3-Stellung substituiert ist, dieser aus einem geradkettigen oder verzweigten Alkylrest mit 4 bis 6 Kohlenstoffatomen besteht, mit Ausnahme der Verbindung 4-Butyl-1-trifluormethylbenzol.

8. Neue durch Fluor enthaltende Gruppen substituierte aromatische Verbindungen der Formel

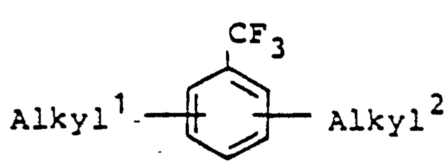

wobei $Alkyl^1$ und $Alkyl^2$ gleich sind und für einen geradkettigen oder verzweigten Kohlenwasserstoffrest mit 3 bis 6 Kohlenstoffatomen stehen oder verschieden sind, wobei $Alkyl^1$ für Methyl oder Ethyl steht und Alkyl einen geradkettigen oder verzweigten Kohlenwasserstoffrest mit 3 bis 6 Kohlenstoffatomen steht.

9. Neue durch Fluor enthaltende Gruppen substituierte aromatische Verbindungen der Formel

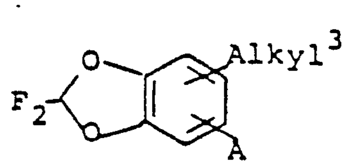

wobei für den Fall, daß A Wasserstoff bedeutet, $Alkyl^3$ für einen geradkettigen oder verzweigten Kohlenwasserstoffrest mit 4 bis 6 Kohlenstoffatomen steht, oder für den Fall, daß A Halogen, Niederalkyl

oder Niederalkoxy bedeutet, $Alkyl^3$ für einen Niederalkylrest steht.

10. Neue durch Fluor enthaltende Gruppen substituierte aromatische Verbindungen der Formel

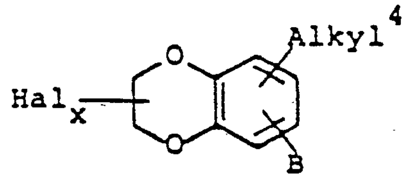

wobei

Hal für Halogen steht,

X eine Zahl von 1 bis 4 bedeutet,

$Alkyl^4$ für den Fall, daß B für Wasserstoff steht, einen geradkettigen oder verzweigten Kohlenwasserstoffrest bedeuten und für den Fall, daß B für Halogen, Niederalkyl oder Niederalkoxy steht, Niederalkyl bedeutet,

mit Ausnahme der Verbindung Trifluor-3,4-dioxyethylentoluol.

**Claims**

1. Process for the preparation of aromatic compounds, which are substituted by radicals containing fluorine, of the formula

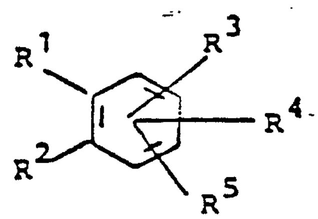

in which

$R^1$ represents a $CF_3$ or $CF_3O$ radical or, together with $R^2$, forms an $-OCF_2O-$, $-O-CHF-CF_2-O-$, $-O-CF_2CFCl-O-$ or $-O-CF_2-CF_2-O-$group,

$R^2$, $R^3$ and $R^4$ are the same or different and denote hydrogen, halogen, hydroxyl, alkyl, aryl, alkoxy or aroxy and

$R^5$ represents an optionally substituted alkyl radical,

characterised in that aromatic compounds of the formula

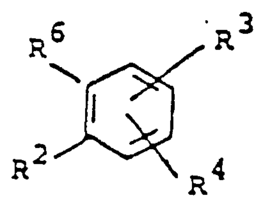

in which

$R^6$ represents a $CF_3$, $CF_2Cl$, $CCl_2F$, $CCl_3$ or $CF_3O$ radical or, together with $R^2$, forms an $-OCF_2O-$, $-O-CHF-CF_2-O-$, $-O-CF_2CFCl-O-$ or $-O-CF_2-CF_2-O-$group, and $R^2$, $R^3$ and $R^4$ have the abovementioned meaning,

are reacted with an alkylating agent in the presence of hydrogen fluoride in the temperature range from 0 to 150 °C.

2. Process according to Claim 1, characterised in that the alkylating agent used is an alkyl halide, alcohol, ether, an olefine, an alkyl aromatic compound or an alkylhalogenoformate.

3. Process according to Claims 1 and 2, characterised in that hydrogen fluoride is employed in an amount larger than the amount corresponding to the halogen present which is not fluorine, which is to be replaced by fluorine during the reaction.

4. Process according to Claims 1 and 2, characterised in that when aromatic compounds of the formula

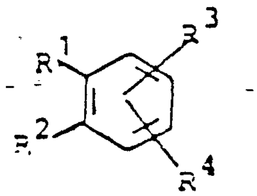

in which $R^1$, $R^2$, $R^3$ and $R^4$ have the meaning indicated in Claim 1, are employed, the hydrogen fluoride is employed in an amount which is sufficient to form a separate phase in the reaction mixture.

5. Process according to Claims 1 to 4, characterised in that the alkylating agent is employed in an amount of 0.1 to 4 mols, relative to 1 mol of the aromatic compound.

6. Process according to Claims 1 to 5, characterised in that the alkylating agent is employed in less than the stoichiometric amount, relative to the aromatic compound, and the unreacted aromatic compound is subjected to further alkylation.

7. New aromatic compounds, substituted by groups containing fluorine, of the formula

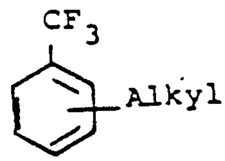

in which, in the case where the alkyl radical is substituted in the 4-position, it consists of a straight-chain or branched hydrocarbon radical having 3 to 6 carbon atoms or, in the case where the alkyl radical is substituted in the 2-position or 3-position, it consists of a straight-chain or branched alkyl radical having 4 to 6 carbon atoms, with the exception of the compound 4-butyl-1-trifluoromethylbenzene.

8. New aromatic compounds, substituted by groups containing fluorine, of the formula

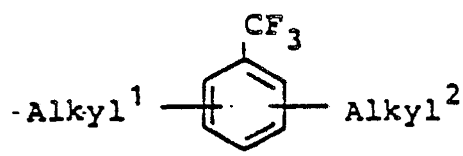

wherein $Alkyl^1$ and $Alkyl^2$ are the same and represent a straight-chain or branched hydrocarbon radical having 3 to 6 carbon atoms or are different, $Alkyl^1$ representing methyl or ethyl and Alkyl representing a straight-chain or branched hydrocarbon radical having 3 to 6 carbon atoms.

9. New aromatic compounds, substituted by groups containing fluorine, of the formula

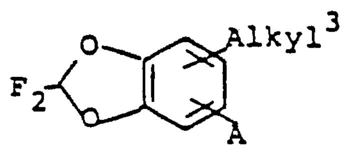

wherein, in the case where A denotes hydrogen, $Alkyl^3$ represents a straight-chain or branched hydrocarbon radical having 4 to 6 carbon atoms, or in the case where A denotes halogen, lower alkyl or lower alkoxy, $Alkyl^3$ represents a lower alkyl radical.

10. New aromatic compounds, substituted by groups containing fluorine, of the formula

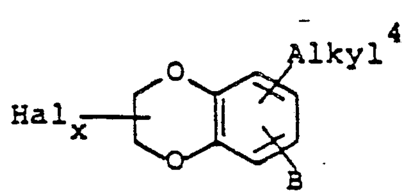

wherein

Hal represents halogen,

X denotes a number from 1 to 4,

$Alkyl^4$ in the case where B represents hydrogen, denotes a straight-chain or branched hydrocarbon radical and, in the case where B represents halogen, lower alkyl or lower alkoxy, it denotes lower alkyl,

with the exception of the compound trifluoro-3,4-dioxyethylenetoluene.

**Revendications**

1. Procédé de production de composés aromatiques qui sont substitués par des restes contenant du fluor, de formule

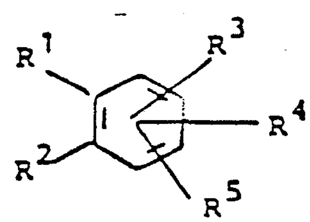

dans laquelle

$R^1$ représente un reste $CF_3$ ou $CF_3O$ ou forme conjointement avec $R^2$ un groupe $-OCF_2O-$, $-O-CHF-CF_2-O-$, $-O-CF_2CFCl-O-$ ou $-O-CF_2-CF_2-O-$,

$R^2$, $R^3$ et $R^4$ sont identiques ou différents et représentent l'hydrogène, un halogène, un groupe hydroxy, alkyle, aryle, alkoxy ou aroxy, et

$R^5$ est un reste alkyle éventuellement substitué,

caractérisé en ce qu'on fait réagir des composés aromatiques de formule

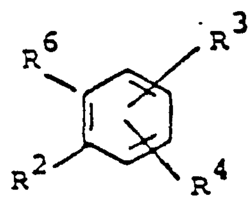

dans laquelle

$R^6$ est un reste $CF_3-$, $CF_2Cl-$, $CCl_2F-$, $CCl_3-$ ou $CF_3O-$ ou forme conjointement avec $R^2$ un groupe $-OCF_2O-$, $-O-CHF-CF_2-O-$, $-O-CF_2CFCl-O-$ ou $-O-CF_2-CF_2-O-$ et $R^2$, $R^3$ et $R^4$ ont la définition donnée ci-dessus,

avec un agent alkylant en présence de fluorure d'hydrogène dans l'intervalle de température de 0 à 150 °C.

2. Procédé suivant la revendication 1, caractérisé en ce qu'on utilise comme agent alkylant un halogénure d'alkyle, un alcool, un éther, une oléfine, un composé alkylaromatique ou un ester alkylique d'acide halogénoformique.

3. Procédé suivant les revendications 1 et 2, caractérisé en ce qu'on utilise le fluorure d'hydrogène en une quantité plus grande que n'y correspond la quantité de l'halogène non présent comme fluorure qui doit être remplacée par du fluor au cours de la réaction.

4. Procédé suivant les revendications 1 et 2, caractérisé en ce que lors de l'utilisation de composés aromatiques de formule

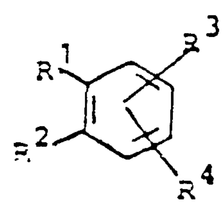

dans laquelle $R^1$, $R^2$, $R^3$ et $R^4$ ont la définition mentionnée dans la revendication 1, on utilise le fluorure d'hydrogène en une quantité qui est suffisante pour former une phase séparée dans le mélange réactionnel.

5. Procédé suivant les revendications 1 à 4, caractérisé en ce qu'on utilise l'agent alkylant en une quantité de 0,1 à 4 moles pour 1 mole du composé aromatique.

6. Procédé suivant les revendications 1 à 5, caractérisé en ce qu'on utilise l'agent alkylant en quantité déficitaire par rapport au composé aromatique et on amène à une nouvelle alkylation le composé aromatique n'ayant pas réagi.

7. Composés aromatiques nouveaux substitués par des groupes contenant du fluor, de formule

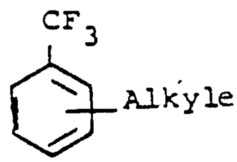

dans laquelle au cas où le reste alkyle est substitué en position 4, ce reste consiste en un reste hydrocarboné à chaîne droite ou à chaîne ramifiée ayant 3 à 6 atomes de carbone ou bien au cas où le reste alkyle est substitué en position 2 ou 3, ce reste est constitué par un reste alkyle à chaîne droite ou à chaîne ramifiée ayant 4 à 6 atomes de carbone, à l'exception du 4-butyl-1-trifluorométhylbenzène.

8. Composés aromatiques nouveaux substitués par des groupes contenant du fluor, de formule

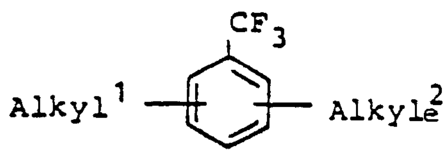

dans laquelle Alkyle$^1$ et Alkyle$^2$ sont identiques et représentent un reste hydrocarboné à chaîne droite ou à chaîne ramifiée ayant 3 à 6 atomes de carbone ou sont différents, auquel cas Alkyle$^1$ représente un groupe méthyle ou éthyle et Alkyle$^2$ représente un reste hydrocarboné à chaîne droite ou à chaîne ramifiée ayant 3 à 6 atomes de carbone.

9. Composés aromatiques nouveaux substitués par des groupes contenant du fluor, de formule

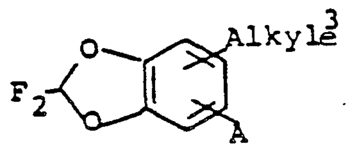

dans laquelle au cas où A est un atome d'hydrogène, Alkyle$^3$ est un reste hydrocarboné à chaîne droite ou à chaîne ramifiée ayant 4 à 6 atomes de carbone ou bien, au cas où A est un halogène, un groupe alkyle inférieur ou un groupe alkoxy inférieur, Alkyle$^3$ représente un reste alkyle inférieur.

10. Composés aromatiques nouveaux substitués par des groupes contenant du fluor, de formule

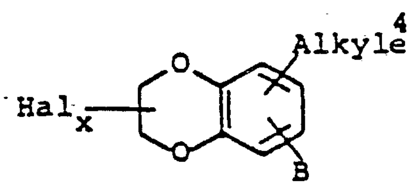

dans laquelle

Hal représente un halogène,

X est un nombre de 1 à 4,

Alkyle$^4$ désigne un reste hydrocarboné à chaîne droite ou à chaîne ramifiée au cas où B est un atome d'hydrogène, et désigne un groupe alkyle inférieur au cas où B représente un halogène, un groupe alkyle inférieur ou un groupe alkoxy inférieur,

à l'exception du trifluoro-3,4-dioxyéthylène-toluène.